# EUROPEAN PATENT APPLICATION

(11) **EP 1 676 573 A1**
(43) Date of publication of application: **05.07.2006**
(21) Application number: 04380287.5
(22) Date of filing: 30.12.2004
(51) Int. Cl.: A61K 31/40, A61K 31/4184, A61K 31/17, A61K 31/404, A61K 31/519, A61K 31/185, A61P 15/10

(54) **Phamaceutical composition comprising a 2,5-dihydroxybenzenesulfonic-compound, a potassium ion channel modulator and a phosphodiesterase type 5 inhibitor**

(71) Applicant: LABORATORIOS DEL DR. ESTEVE, S.A., 08041 Barcelona (ES)
(72) Inventor: Saenz de Tejada Gorman, Iñigo, 28034 Madrid (ES)
(74) Representative: Carvajal y Urquijo, Isabel

(57) **Abstract**

The present invention relates to a pharmaceutical composition comprising a 2,5-dihydroxybenzenesulfonic compound, a potassium ion channel modulator and a phosphodiesterase type 5 inhibitor, a medicament comprising said pharmaceutical composition as well as the use of said pharmaceutical composition for the manufacture of a medicament.

## Description

The present invention relates to a pharmaceutical composition comprising a 2,5-dihydroxybenzenesulfonic compound, a potassium ion channel modulator and a phosphodiesterase type 5 inhibitor, a medicament comprising said pharmaceutical composition as well as the use of said pharmaceutical composition for the manufacture of a medicament.

Sexual disfunction is a significant clinical problem that can affect both males and females. The causes of sexual disfunction are diverse and usually include organic as well as physiological factors. Organic causes are often related to underlying vascular diseases such as those associated with hypertension or diabetes mellitus.

Current methods for treating sexual disfunctions are frequently based on the administration of an active substance that inhibits activity of the enzyme phoshodiesterase type 5. Typical examples of such inhibitors include sildenafil, Vardenafil, Tadalafil and the like. However, due to the occurence of undesired side effects such as severe headaches or cardiovascular problems like heart attacks their use is limited. Moreover, these usual treatment methods often do not give satisfactory results especially for diabetic patients.

It was therefore an object of the present invention to provide a medicament that is suitable for the prophylaxis and/or treatment of sexual disfunction, which preferably does not show the undesired side effects of known medicaments, or at least less frequent and/or less pronounced.

It has surprisingly been found that the pharmacological efficacy of a phosphodiesterase type 5 inhibitor may be enhanced by its administration in combination with one or more 2,5-dihydroxybenzenesulfonic compounds of general formula I given below and one or more potassium ion (K⁺) channel modulators.

Since these three components interact synergistically, the individual doses of the phosphodiesterase type 5 inhibitor like that of the other two components may be reduced and fewer and/or less pronounced to none undesired side effects occur.

Consequently, the inventive pharmaceutical composition is particularly suitable for diabetics and patients with cardiovascular diseases who often suffer from severe side from a higher dose of any of the individual components (A), (B) or (C).

Thus, in one of its aspects the present invention relates to a pharmaceutical composition consisting of components (A), (B) and (C), wherein
(A) is at least one 2,5-dihydroxybenzenesulfonic compound of general formula I, 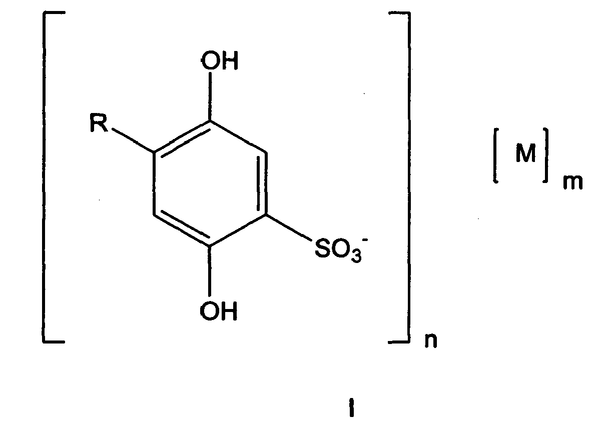 wherein
   R represents H or SO₃⁻,
   M represents at least one cation,
   n represents 1 or 2,
   m represents 1 or 2,
   optionally in form of a pharmaceutically acceptable solvate,
(B) is at least one potassium ion (K⁺) channel modulator, and
(C) is at least one phosphodiesterase type 5 inhibitor (PDE5 inhibitor).

The cation M in the 2,5-dihydroxybenzenesulfonic compounds of general formula I of component (A) may be any physiologically acceptable cation known to those skilled in art, e.g. from P. Heinrich Stahl, Camille G. Wermuth (Editiors), "Handbook of Pharmaceutical Salts - Properties, Selections and Use", Verlag Helvetica Chimica Acta, Zürich, Switzerland, Wiley-VCH, Weinheim, Germany, 2002. The respective literature description is hereby incorporated by reference and is part of the disclosure. Those skilled in the art understand that the cation M has to be chosen in such a way that the overall charge of the 2,5-dihydroxybenzenesulfonic compounds of general formula I is neutral.

The present invention encompasses also the use of a mixture of at least two of the afore mentioned 2,5-dihydroxybenzenesulfonic compounds of general formula I as well as mixed salts of these compounds, i.e. compounds with different cations M and/or different 2,5-dihydroxybenzenesulfonic residues.

Preferably the cation(s) M of the 2,5-dihydroxybenzenesulfonic compounds of general formula I is (are) selected from the group consisting of Ca²⁺, Mg²⁺, Na⁺, K⁺ and [NH₄₋ₓRₓ]⁺, wherein x is 0, 1, 2, 3 or 4 and R represents a branched or unbranched C₁₋₄-alkyl-radical. If x is greater than 1, i.e. if two or more alkyl-radicals are present in the [NH₄₋ₓRₓ]⁺-cation, they may be identical or different, whereby identical alkyl-radicals may be preferred.

Preferably the pharmaceutical composition of the present invention may comprise one or more compounds selected from the group consisting of calcium 2,5-dihydroxybenzenesulfonate (calcium dobesilate), diethylamine 2,5-dihydroxybenzenesulfonate (ethamsylate) and bis(diethylamine)-2,5-dihydroxybenzene-1,4-disulfonate (diethylamine persilate). Particularly preferably calcium 2,5-dihydroxybenzenesulfonate (calcium dobesilate) is used as component (A) in the pharmaceutical composition according to the present invention.

The inventively used 2,5-dihydroxybenzenesulfonate compounds of general formula I may also be in the form of solvates, particularly in the form of hydrates. The manufacture of the 2,5-dihydroxybenzenesulfonate compounds of general formula I as well as their solvates may be accomplished by the use of reagents and methods known to those skilled in the art.

The manufacture of calcium 2,5-dihydroxybenzenesulfonate (calcium dobesilate) and diethylamine 2,5-dihydroxybenzenesulfonate (ethamsilate) is known, for example, from "The Merck Index"-13^{th} edtion, Merck & Co., R. Rahway, N.J., USA, 2001. Said literature description is hereby incorporated by reference and is part of the disclosure. The manufacture of bis(diethylamine) 2,5-dihydroxybenzene-1,4-disulfonate (diethylamine persilate) is known, for example, from French Patent FR 73/17709 (Publication No. 2,201,888). The respective description is hereby incorporated by reference and is part of the disclosure.

It is well known to those skilled in the art that different types and subtypes of K⁺ channels exist, e.g. from Christopher G. Sobey "Potassium Channel Function in Vascular Disease, Arterioscler. Throm. Vasc. Biol., January 2001, pages 28 ff, which is hereby incorporated by reference and forms part of the disclosure. Generally different K⁺ channel modulators show different activity for the different K⁺ channels. It can be tested by methods known to those skilled in the art, for which K⁺ channel a certain K⁺ channel modulator shows the best activity.

Preferably, the K⁺ channel modulator according to component (B) of the inventive pharmaceutical composition may be a K⁺ channel opener, more preferably a large conductance Ca(2+) activated K⁺ channel opener (activator). K⁺ channel openers including large conductance Ca(2+) activated K⁺ channel openers (activators) that may be used as component (B) as well as methods for their preparation are well known to those skilled in the art. Large conductance Ca(2+) activated K⁺ channel openers (activators) are also known as bk-channel openers and include the compounds NS-1619, NS-8, NS-1608, NS-0004 and BMS-204352.

According to the present invention a potassium ion (K⁺) channel opener (activator) is a compound having an IC₅₀ value of preferably < 500 µM, more preferably < 100 µM, yet more preferably 0,1 nM - < 50 nM, most preferably 1nM - < 10 nM as determined according to the method described in the publication of Holland et al., Br. J. Pharmacol. 1996, 117(1), pages 119-29. The respective part of the description is hereby incorporated by reference and forms part of the present disclosure.

Preferably the inventive pharmaceutical composition comprises one or more K⁺ channel openers selected from the group consisting of benzimidazole derivatives of general formula I, 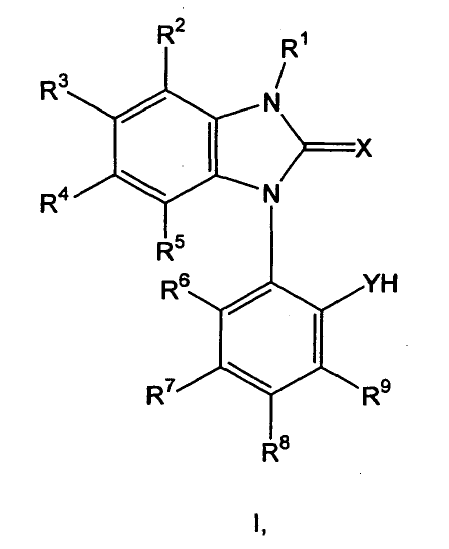 wherein
X represents O, S or NCN,
Y represents O or S,
R¹ represents hydrogen, NH₂ or branched or unbranched C₁₋₆-alkyl,
R², R³, R⁴, R⁵ are each independently selected from the group consisting of hydrogen, halogen, CF₃, NO₂, NH₂, OH, C₁₋₆-alkoxy, C(=O)-phenyl or SO₂NR^{A}R^{B}, wherein R^{A} and R^{B}, identical or different, represent H or C₁₋₆-alkyl,
R⁶ represents hydrogen or NO₂,
R⁷ represents hydrogen, halogen, phenyl, CF₃ or NO₂, or
R⁸ represents hydrogen or NO₂,
or
R⁶ and R⁷ or R⁷ and R⁸ together with the two bridging carbon atoms from the phenyl ring form a C₄₋₇ carbocyclic ring, which may be saturated, unsaturated or aromatic,
R⁹ is hydrogen, halogen, NO₂ or SO₂NR^{A}R^{B}, wherein R^{A} and R^{B}, identical or different represent hydrogen or C₁₋₆-alkyl,
optionally in the form of a corresponding salt, or a corresponding solvate thereof,
and/or one or more compounds selected from the group consisting of 6-Amino-1,2-dihydro-1-hydroxy-2-imino-4-piperidinopyrimidine (minoxidil), (R)-(-)-2-[4-(4-Methyl-6-oxo-1,4,5,6,-tetrahydropyridazin-3-yl)phenylhydrazono]propanedinitrile (levosimendan), N-[2-Amino-4-(4-fluorobenzylamino)phenyl]carbamic acid ethyl ester (retigabine), (-)-3-[5-oxo-2-(trifluoromethyl)-1,4,5,6,7,8-hexahydroquinolin-4(S)-yl]benzonitrile (ZD-0947), 2-Amino-5-(2-fluorophenyl)-4-methyl-1H-pyrrole-3-carbonitrile (NS-8), (3S, 4R)-3-Hydroxy-2,2-dimethyl-4-(2-oxopiperidin-1-yl)-N-phenyl-1-benzopyran-6-sulfonamide (KCO-912), (6-Chloro-3-(1-methylcyclopropylamino)-4H-thieno[3,2-e][1,2,4]thiadiazine-1,1-dioxide (NN-414), ABT-598, iptakalim hydrochloride, pinacidil, cromakalin, levcromakalin, aprikalim, N-(2-Hydroxyethyl)pyridine-3-carboxamide nitrate ester (nicorandil), (±)-(5-chloro-2-methoxyphenyl)-1,3-dihydro-3-fluoro-6-(trifluoromethyl)-2H-indol-2-one and ((3S)-(+)-(5-chloro-2-methoxyphenyl)-1,3-dihydro-3-fluoro-6-(trifluoromethyl)-2H-indol-2-one (also known as BMS-204352).

More preferably the K⁺ channel opener of component (B) may be selected from the group consisting of NS-1619, NS-8, NS-1608, NS-0004 and BMS-204352, optionally in form of a salt or a corresponding solvate, which are all Conductance Ca(2+) activated K⁺ channel activators. Yet more preferably component (B) may be selected from the group consisting of NS-1619 and NS-8, optionally in form of salt or a corresponding solvate. Most preferably component (B) is NS-1619, optionally in form of a salt or a corresponding solvate.

The manufacture of the afore mentioned potassium ion channel openers is well known to those skilled in the art, e.g. for the benzimidazole derivatives from EP 0 477 818, which is hereby incorporated by reference and forms part of the disclosure. The compound NS-8 (2-amino-5-(2-fluorophenyl)-4-methyl-1H-pyrrole-3-carbonitrile or 2-amino-3-cyano-5-(2-fluorphenyl)-4-methylpyrrole) and its preparation 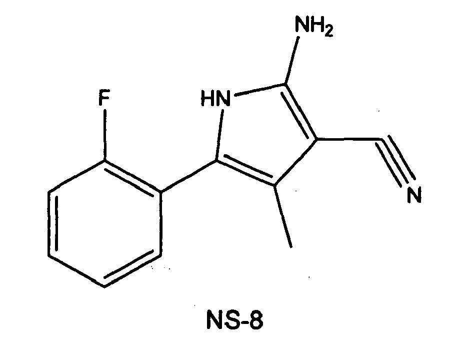 is for example described in WO 99/61016, AU9939538, EP1 283 040, EP1 369 416, WO96/40634, EP 842 923, WO99/36068 and EP1 057 485. The respective parts of the descriptions are hereby included by reference and form part of the disclosure.

The compound NS-1619 (5-(trifluoromethyl)-1-(5-(trifluoromethyl)-2-hydroxyphenyl)-1 H-benzo[d]imidazol-2(3H)-one or 1,3-dihydro-I-[2-hydroxy5 (trifluoromethyl) phenyl] 5- (trifluoromethyl) 2-Hbenimidazol-one) having the following structure 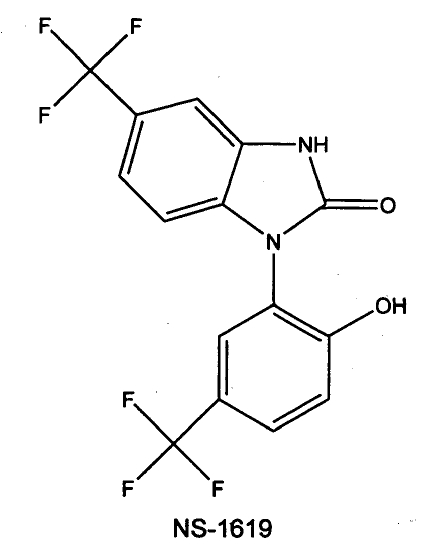 as well as the compound NS-1608 (1-(5-chloro-2-hydroxyphenyl)-3-(3-(trifluoromethyl)phenyl)urea) 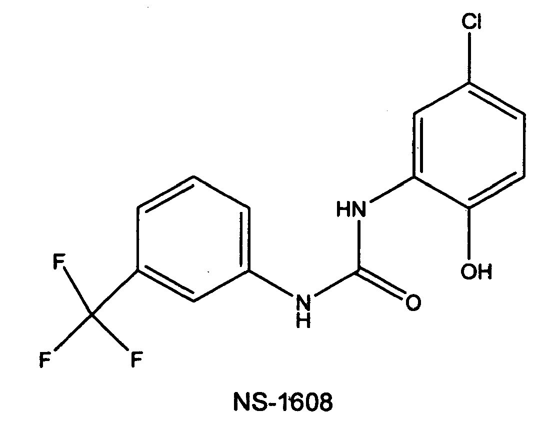 and its analogue NS-0004are also well known to those skilled in the art.

According to the present invention a PDE5 inhibitor is a compound having an IC₅₀ value of preferably < 500 µM, more preferably < 100 µM, yet more preferably 0,1 - 50 nM, most preferably 0,1 - 10 nM as determined according to the method described in the publication of Ballard et al., J. Urol. 1998, 159(6), pages 2164-71. The respective part of the description is hereby incorporated by reference and forms part of the present disclosure.

The phosphodiesterase type 5 inhibitor according to component (C) of the inventive pharmaceutical composition may preferably be selected from the group consisting of sildenafil, vardenafil, tadalafil and dipyridamole, in each case optionally in form of a physiologically acceptable salt, or a corresponding solvate thereof.

Preferably component (C) of the inventive pharmaceutical composition may be selected from the group consisting of sildenafil, sildenafil hydrochloride, sildenafil nitrate, sildenafil acetate, sildenafil hydrobromide, sildenafil lactate, sildenafil mesilate, sildenafil maleate, sildenafil fumarate, sildenafil succinate, sildenafil tatrate, sildenafil ascorbate and sildenafil citrate.

More preferably component (C) may be selected from the group consisting of sildenafil and sildenafil citrate.

PDE5 Inhibitors, in particular sildenafil (1-[[3-(4,7-Dihydro-1-methyl-7-oxo-3-propyl-1*H*-pyrazolo[4,3-*d*]pyrimidin-5-yl)-4-ethoxyphenyl]sulfonyl]-4-methylpiperazine), vardenafil (2-[2-Ethoxy-5-(4-ethyl-piperazine-1-sulfonyl)-phenyl]-5-methyl-7-propyl-3H-imidazo[5,1-f][1,2,4]triazin-4-one), tadalafil (6-Benzo[1,3]dioxol-5-yl-2-methyl-2,3,6,7,12,12a-hexahydro-pyrazino[1',2':1,6]pyrido[3,4-b]indole-1,4-dione) and dipyridamole (2-[{6-[Bis-(2-hydroxy-ethyl)-amino]-4,8-di-piperidin-1-yl-pyrimido[5,4-d]pyrimid in-2-yl}-(2-hydroxy-ethyl)-amino]-ethanol), their salts and corresponding solvates are well known to those skilled in the art. For example, sildenafil and its preparation are described in US 5,250,534, EP 463,756 and EP 916 675. The respective parts of the description are hereby incorporated by reference and form part of the present disclosure.

Another aspect of the present invention relates to a pharmaceutical composition according to the present invention, wherein one or more of the components A, B and C, preferably the PDE5-Inhibitor is present in a sub therapeutic dose. Surprisingly such a combination is highly active, even though such an amount of the respective single component - if administered alone - does not show a significant pharmaceutical activity.

Preferred is an inventive pharmaceutical composition comprising
(A) at least one compound selected from the group consisting of calcium dobesilate, ethamsylate and diethylamine persilate, optionally in form of a corresponding solvate,
(B) at least one compound selected from the group consisting of NS-1619, NS-8, NS-1608, NS-0004 and BMS-204352, optionally in form of a salt or a corresponding solvate thereof, and
(C) at least one compound selected from the group consisting of sildenafil, vardenafil, tadalafil and dipyridamole, optionally in form of a salt or a corresponding solvate thereof.

Particularly preferred is an inventive pharmaceutical composition comprising
(A) calcium dobesilate, optionally in form of a corresponding solvate,
(B) NS-1619, optionally in form of a salt or a corresponding solvate thereof, and
(C) sildenafil, optionally in form of a salt, preferably sildenafil citrate, or a corresponding solvate thereof.

The term "salt" is to be understood as meaning any form of an active compound present in the inventive pharmaceutical composition in which said compound assumes an ionic form or is charged and is coupled with a counter-ion (a cation or anion) or is in solution. By this are also to be understood complexes of the active compound with other molecules and ions, in particular complexes which are complexed via ionic interactions.

The term "physiologically acceptable salt" is understood in particular, in the context of this invention, as salt (as defined above) formed either with a physiologically tolerated acid, that is to say salts of the particular active compound with inorganic or organic acids which are physiologically tolerated - especially if used on humans and/or mammals - or with at least one, preferably inorganic, cation which are physiologically tolerated - especially if used on humans and/or mammals. Examples of physiologically tolerated salts of particular acids are salts of: hydrochloric acid, hydrobromic acid, sulfuric acid, hydrobromide, monohydrobromide, monohydrochloride or hydrochloride, methiodide, methanesulfonic acid, formic acid, acetic acid, oxalic acid, succinic acid, malic acid, tartaric acid, mandelic acid, fumaric acid, lactic acid, citric acid, glutamic acid, hippuric acid picric acid and/or aspartic acid. Examples of physiologically tolerated salts of particular bases are salts of alkali metals and alkaline earth metals and with [NH₄₋ₓRₓ]⁺-lons, wherein x is 0, 1, 2, 3 or 4 and the Rs independent from one another represent a branched or unbranched C₁₋₄₋alkyl-radical.

The term "solvate" according to this invention is to be understood as meaning any form of an active compound used in the pharmaceutical composition of the present invention in which this compound has attached to it via non-covalent binding another molecule, e.g. a polar solvent molecule, especially including hydrates and alcoholates, e.g. methanolate.

The daily dosage for mammals, especially humans of any of the active substances of the inventive pharmaceutical composition may vary depending on factors that have their basis in the respective species or other factors, such as age, sex, weight or degree of illness and so forth. The daily dosage may be administered to the patient via one or several intakes per day.

Preferably the pharmaceutical composition according to the present invention is suitable for once daily administration and comprises component (A) in an amount of 0.5 - 40 mg/kg, component (B) in an amount of 0.005 - 20 mg/kg and component (C) in an amount of 0.005 - 2 mg/kg,
more preferably the pharmaceutical composition according to the present invention is suitable for once daily administration and comprises component (A) in an amount of 2.5 - 20 mg/kg, component (B) in an amount of 0.075 - 10 mg/kg and component (C) in an amount of 0.075 - 0.6 mg/kg,
yet more preferably the inventive active substance combination comprises component (A) in an amount of 5 - 15 mg/kg, component (B) in an amount of 0.15 - 7.5 mg/kg and component (C) in an amount of 0.15 - 0.45 mg/kg. All of the amounts are given in mg/kg body weight.

Also preferably the pharmaceutical composition according to the present invention is suitable for once daily administration and comprises component (A) in an amount of 10-2000 mg, preferably 50-1500 mg, more preferably 100-1000 mg, most preferably 150-750 mg, component (B) in an amount of 1-1000 mg, preferably 2.5-500 mg, more preferably 5-375 mg, most preferably 7.5-150 mg, and component (C) in an amount of 1-150 mg, preferably 2.5-100 mg, more preferably 5-50 mg, most preferably 7.5-30 mg.

The inventive pharmaceutical composition may also be characterized via its effect on erectile response induced by cavernosal nerve electrical stimulation in anesthetized diabetic rats, as described in the reference of Saénz de Tejada et al., International Journal of Impotence Research 2003, 15, 90-93 under "Methods - Erectile responses to cavernosal nerve stimulation in anaesthetized rats". The respective part of the description is hereby incorporated by reference and forms part of the disclosure.

An inventive pharmaceutical composition may be preferred, wherein the combined intravenous administration of 10 mg/kg body weight of component (A), -0.3 mg /kg body weight of component (B) and 0.3 mg/kg body weight of component (C) causes an increase in intracavernosal pressure (ICP) in anesthetized diabetic rats of > 3000 area under curve, mm Hg x s, preferably of > 3500 area under curve, mm Hg x s, more preferably of > 3750 area under curve, mm Hg x s, yet more preferably of > 4000 area under curve, mm Hg x s, most preferably of > 4500 area under curve, mm Hg x s, in each case measured at a frequency of 3 Hz.

Another aspect of the present invention relates to a medicament comprising an inventive pharmaceutical composition and optionally one or more auxiliary substances.

Said inventive medicament is suitable for the prophylaxis and/or treatment of one or more disorders selected from the group consisting of sexual disfunction in man such as impotence or erectile disfunction, sexual disfunctions in woman, hypertension, type I diabetes mellitus, type II diabetes mellitus, hypercholesterolemia, bladder instability, urinary incontinence, asthma, ischemic injury, ischemic insufficiency to the brain, cardiovascular diseases, preterm labor, labor preparatory to Caesarean delivery, alopecias, epilepsy, gastrointestinal disorders including ulcers and dyspepsia, spasms such as gastrointestinal spasms, inflammatory diseases such as gastrointestinal inflammation and cancer.

The indication urinary incontinence includes also the indications imperative micturition (urge incontinence), hyperreflexia, urinary stress incontinence, mixed incontinence and Enuresis as well as others known to those skilled in the art.

For a more detailed description of these definitions and a standardisation of terminology reference is made to Abrams et al, Neurology and Urodynamics 21:167-178 (2002). The respective part of the description is hereby incorporated by reference and forms part of the disclosure.

Preferably the inventive medicament may be used for the prophylaxis and/or treatment of sexual disfunction in man such as erectile disfunction and/or impotence, and/or sexual disfunction in woman.

More preferably the inventive medicament may be used for the prophylaxis and/or treatment of any of the afore mentioned disorders that are at least partially caused by type I or type II diabetes mellitus, particularly sexual disfunction in man such as erectile disfunction and/or impotence, and/or sexual disfunction in woman.

Most preferably the inventive medicament may be used for the prophylaxis and/or treatment of sexual disfunction in man such as erectile disfunction and/or impotence, and/or sexual disfunction in woman that are at least partially caused by type I or type II diabetes mellitus.

Another aspect of the present invention relates to the use of an inventive Pharmaceutical composition for the prophylaxis and/or treatment of one or more disorders selected from the group consisting of sexual disfunctions in man, sexual disfunctions in woman, hypertension, type I diabetes mellitus, type II diabetes mellitus, hypercholesterolemia, bladder instability, urinary incontinence, asthma, ischemic injury, ischemic insufficiency to the brain, cardiovascular diseases, preterm labor, for stopping labor preparatory to Caesarean delivery, alopecias, epilepsy, gastrointestinal disorders including ulcers and dyspepsia, spasms, preferably gastrointestinal spasms, inflammatory diseases, preferably gastrointestinal inflammation and cancer.

Those skilled in the art understand that the components (A), (B) and (C) of the inventive pharmaceutical composition may be administered simultaneously or sequentially to one another, whereby the respective components may be administerd via the same or different administration pathways, e.g. orally, parenterally or transdermally. Preferably all components (A), (B) and (C) are administered simultaneously in one and the same administration form.

Yet another aspect of the present invention relates to pharmaceutical formulations in different pharmaceutical forms comprising an inventive pharmaceutical composition and optionally one or more auxiliary substances.

The pharmaceutical formulations may also contain one or more auxiliary substances known to those skilled in the art. The selection of these auxiliary substances depends on the route of administration.

The pharmaceutical formulations according to the present invention may be produced according to standard procedures known to those skilled in the art, e.g. from the tables of contents from "Pharmaceutics: the Science of Dosage Forms", Second Edition, Aulton, M.E. (Ed.) Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 and "The Theory and Practice of Industrial Pharmacy", Lachman L., Lieberman H. and Kanig J. (Eds.), Lea & Febiger, Philadelphia (1986). The respective descriptions are incorporated by reference and are part of the disclosure.

Suitable routes of administration for the inventive pharmaceutical composition include parenteral administration such as intravenous, subcutaneous or intramuscular administration, sublingual administration, bucal administration, transdermal administration and oral administration. The administration via a suppository constitutes another possible route of administration.

In a preferred embodiment of the present invention, the pharmaceutical formulation is suitable for oral administration.

If the pharmaceutical formulation is suitable for oral administration, it may preferably be in the form of a tablet, a pill, a powder, a capsule, a solution or a suspension.

The pharmaceutical formulation of the present invention for oral administration may also be in the form of multiparticulates, preferably pellets or granules, optionally compressed into a tablet, filled into a capsule or suspended in a suitable liquid. Suitable liquids are known to those skilled in the art.

Suitable tabletting auxiliary substances include excipients such as sodium citrate, calcium carbonate or calcium phosphate, disintegrants such as starch or silicates, binding agents such as polyvinylpyrrolidone, sucrose, gelatin or acacia, lubricating agents such as magnesium stearate, sodium lauryl sulfate or talc. Solid compositions on the basis of such ingredients may also be employed as filers in soft and hard-filled gelatin capsules. Other materials that may be used for this purpose include lactose or milk sugar as well as high molecular weight polyethylene glycols.

When suspensions or solutions are desired for oral administration they are preferably based on water and may include auxiliary substances such as sweetening agents, flavoring agents, coloring agents, emulsifying agents and/or suspending agents. Diluents such as ethanol, propylene glycol, glycerin alone, in combinations thereof, optionally in a mixture with water may also be used.

For purposes of parenteral administration, solutions in sesame or peanut oil or in aqueous propylene glycol may be employed, as well as sterile aqeuos solutions or suspensions of the active substances. Such aqueous solutions may be suitably buffered, if necessary, and the liquid diluent first rendered isotonic with sufficient saline or glucose. Such aqueous solutions are especially suitable for intravenous, intramuscular, subcutaneous or intraperitoneal injection purposes.

For purposes of transdermal, for example topical, administration dilute sterile, aqeous or partially aqueous solutions are usually employed.

In one preferred embodiment of the present invention the pharmaceutical formulation may comprise at least one of the components (A), (B) and (C) at least partially in a sustained-release form.

By incorporating one or more of these components at least partially or completely in a sustained-release form it is possible to extend the duration of their effect, allowing for the beneficial effects of such a sustained release form, e.g. the maintenance of even concentrations in the blood.

Suitable sustained-release forms as well as materials and methods for their preparation are known to those skilled in the art, e.g. from the tables of contents from "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000);"Controlled Drug Delivery", Vol. I, Basic Concepts, Bruck, S.D. (Ed.), CRC Press Inc., Boca Raton (1983) and from Takada, K. and Yoshikawa, H., "Oral Drug delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encylopedia of Controlled Drug Delivery, Mathiowitz, E. (Ed.), John Wiley & Sons, Inc., New York (1999), Vol. 2, 698-728. The respective descriptions are incorporated by reference and are part of the disclosure.

If the pharmaceutical formulation according to the present invention comprises one or more of the components (A), (B) and (C) in a sustained-release form, said sustained release may preferably be achieved by the application of at least one coating or provision of a matrix comprising at least one sustained-release material.
The sustained-release material is preferably based on an optionally modified, water-insoluble, natural, semisynthetic or synthetic polymer, or a natural, semisynthetic or synthetic wax or fat or fatty alcohol or fatty acid, or on a mixture of at least two of these afore mentioned components.

The water-insoluble polymers used to produce a sustained-release material are preferably based on an acrylic resin, which is preferably selected from the group of poly(meth)acrylates, particularly preferably poly(C₁₋₄)alkyl (meth)acrylates, poly(C₁₋₄)dialkylamino(C₁₋₄)alkyl (meth)acrylates and/or copolymers or mixtures thereof, and very particularly preferably copolymers of ethyl acrylate and methyl methacrylate with a monomer molar ratio of 2:1 (Eudragit NE30D®), copolymers of ethyl acrylate, methyl methacrylate and trimethylammonium ethyl methacrylate-chloride with a monomer molar ratio of 1:2:0.1 (Eudragit RS®), copolymers of ethyl acrylate, methyl methacrylate and trimethylammonium ethyl methacrylate-chloride with a monomer molar ratio of 1:2:0.2 (Eudragit RL®), or a mixture of at least two of the above-mentioned copolymers. These coating materials are commercially available as 30 wt.% aqueous latex dispersions, i.e. as Eudragit RS30D®, Eudragit NE30D® or Eudragit RL30D®, and may also be used as such for coating purposes.

In another embodiment, the sustained-release material is based on water-insoluble cellulose derivatives, preferably alkyl celluloses, particularly preferably ethyl cellulose, or cellulose esters, e.g. cellulose acetate. Aqueous ethyl cellulose dispersions are commercially available, for example, under the trademarks Aquacoat® or Surelease®.

As natural, semisynthetic or synthetic waxes, fats or fatty alcohols, the sustained-release material may be based on carnauba wax, beeswax, glycerol monostearate, glycerol monobehenate, glycerol ditripalmitostearate, microcrystalline wax, cetyl alcohol, cetylstearyl alcohol or a mixture of at least two of these components.

The afore mentioned polymers of the sustained-release material may also comprise a conventional, physiologically acceptable plasticizer in amounts known to those skilled in the art.

Examples of suitable plasticizers are lipophilic diesters of a C₆-C₄₀ aliphatic or aromatic dicarboxylic acid and a C₁-C₈ aliphatic alcohol, e.g. dibutyl phthalate, diethyl phthalate, dibutyl sebacate or diethyl sebacate, hydrophilic or lipophilic citric acid esters, e.g. triethyl citrate, tributyl citrate, acetyltributyl citrate or acetyltriethyl citrate, polyethylene glycols, propylene glycol, glycerol esters, e.g. triacetin, Myvacet® (acetylated mono- and diglycerides, C₂₃H₄₄O₅ to C₂₅H₄₇O₇), medium-chain triglycerides (Miglyol®), oleic acid or mixtures of at least two of said plasticizers.
Aqueous dispersions of Eudragit RS® and optionally Eudragit RL® preferably contain triethyl citrate. The sustained-release material may comprise one or more plasticisers in amounts of, for example, 5 to 50 wt.% based on the amount of polymer(s) used.

The sustained-release material may also contain other conventional auxiliary substances known to those skilled in the art, e.g. lubricants, coloured pigments or surfactants.

The pharmaceutical formulation of the present invention may also comprise one or more of the components (A), (B) and (C) covered by an enteric coating, which dissolves as a function of pH. Because of this coating, part or all of the pharmaceutical formulation can pass through the stomach undissolved and the respective component(s) is (are) only released in the intestinal tract. The enteric coating preferably dissolves at a pH of between 5 and 7.5.

The enteric coating may be based on any enteric material known to those skilled in the art, e.g. on methacrylic acid/methyl methacrylate copolymers with a monomer molar ratio of 1:1 (Eudragit L®), methacrylic acid/methyl methacrylate copolymers with a monomer molar ratio of 1:2 (Eudragit S®), methacrylic acid/ethyl acrylate copolymers with a monomer molar ratio of 1:1 (Eudragit L30D-55®), methacrylic acid/methyl acrylate/methyl methacrylate copolymers with a monomer molar ratio of 7:3:1 (Eudragit FS®), shellac, hydroxypropyl methyl cellulose acetate-succinates, cellulose acetate-phthalates or a mixture of at least two of these components, which can optionally also be used in combination with the above-mentioned water-insoluble poly(meth)acrylates, preferably in combination with Eudragit NE30D® and/or Eudragit RL® and/or Eudragit RS®.

The coatings of the pharmaceutical formulations of the present invention may be applied by the conventional processes known to those skilled in the art, e.g. from Johnson, J.L., "Pharmaceutical tablet coating", Coatings Technology Handbook (Second Edition), Satas, D. and Tracton, A.A. (Eds), Marcel Dekker, Inc. New York, (2001), 863-866; Carstensen, T., "Coating Tablets in Advanced Pharmaceutical Solids", Swarbrick, J. (Ed.), Marcel Dekker, Inc. New York (2001), 455-468; Leopold, C.S., "Coated dosage forms for colon-specific drug delivery", Pharmaceutical Science & Technology Today, 2(5), 197-204 (1999), Rhodes, C.T. and Porter, S.C., Coatings, in Encyclopedia of Controlled Drug Delivery. Mathiowitz, E. (Ed.), John Wiley & Sons, Inc., New York (1999), Vol. 1, 299-311. The respective descriptions are incorporated by reference and are part of the disclosure.

In another embodiment, the pharmaceutical formulation of the present invention contains one or more of the components (A), (B) and (C) not only in sustained-release form, but also in its/their non-retarded form. By combination with the immediately released form, a high initial dose can be achieved for the rapid onset of the beneficial effect. The slow release from the sustained release form then prevents the beneficial effect from diminishing. Such a pharmaceutical formulation is particularly useful for the treatment of acute health problems.

This may be achieved, for example, by a pharmaceutical formulation having at least one immediate-release coating comprising at least one of the components (A), (B) and (C) to provide for rapid onset of the beneficial effect after administration to the patient.

### Pharmacological Methods:

The in-vivo activity of the inventive pharmaceutical composition is tested according to the method described in the reference of Saénz de Tejada et al., International Journal of Impotence Research 2003, 15, 90-93 under "Methods - Erectile responses to cavernosal nerve stimulation in anaesthetized rats". The respective part of the description is hereby incorporated by reference and forms part of the disclosure.

The present invention is illustrated below with the aid of examples. These illustrations are given solely by way of example and do not limit the general spirit of the present invention.

### Examples:

### Example 1:

Hard Gelatin Capsule suitable for once daily administration:

| | |
|---|---|
| Calcium dobesilate | 750 mg |
| NS1619 | 22.5 mg |
| Sildenafil | 22.5 mg |
| Cellulose | 23 mg |
| Magnesiumstearate | 7 mg |
| Colloidal silicon dioxide | 5 mg |
| Total weight | 830 mg |

Calcium dobesilate, NS1619, Sildenafil, Cellulose, Magnesiumstearate and Colloidal silicon dioxide in the afore mentioned amounts were thoroughly mixed in a conventional mixer and then filled into a conventional hard gelatin capsule.

### Pharmacological Methods and Data:

The erectile response to cavernosal nerve electrical stimulation in anaesthetized diabetic rats was determined as described above.

It has been found that the intravenous administration of calcium dobesilate (10 mg/kg body weight), of the K⁺ channel opener NS1619 (0.3 mg/kg or 5 mg/kg body weight) or of the PDE5 inhibitor sildenafil (0.3 mg/kg body weight) - if administered alone - did not significantly modify the erectile response in diabetic rats.

The intravenous administration of inventive pharmaceutical compositions comprising calcium dobesilate (10 mg/kg body weight), NS1619 (0.3 mg/kg or 5 mg/kg body weight) and sildenafil (0.3 mg/kg body weight) caused a significant improvement of the erectile response in diabetic rats. A synergistic effect is found for the said inventive pharmaceutical compositions.

## Claims

1. Pharmaceutical composition comprising
(A) at least one 2,5-dihydroxybenzenesulfonic compound of general formula I, 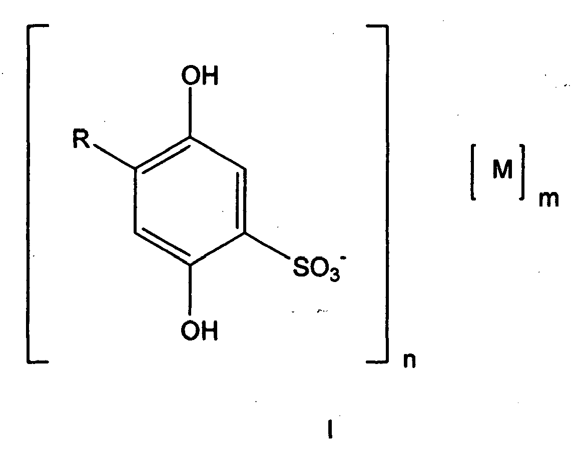 wherein
R represents H or SO₃⁻,
M represents at least one cation,
n represents 1 or 2,
m represents 1 or 2,
optionally in form of a pharmaceutically acceptable solvate,
(B) at least one potassium ion (K⁺) channel modulator, and
(C) at least one phosphodiesterase type 5 inhibitor.

2. Pharmaceutical composition according to claim 1, **characterised in that** the cation(s) M is (are) selected from the group consisting of Ca²⁺, Mg²⁺, Na⁺, K⁺ and [NH₄₋ₓRₓ]⁺, whereby x is 0, 1, 2, 3 or 4 and R represents a branched or unbranched C₁₋₄-alkyl-radical that may be the same or different for x >1.

3. Pharmaceutical composition according to claim 1 or 2, **characterized in that** the compound of general formula I is selected from the group consisting of calcium 2,5-dihydroxybenzenesulfonate (calcium dobesilate), diethylamine 2,5-dihydroxybenzenesulfonate (ethamsylate) and bis(diethylamine)-2,5-dihydroxybenzene-1,4-disulfonate (diethylamine persilate).

4. Pharmaceutical composition according to claim 3, **characterized in that** the compound of general formula I is calcium dobesilate.

5. Pharmaceutical composition according to one or more of claims 1-4, **characterized in that** the modulator of component (B) is a K⁺ channel opener.

6. Pharmaceutical composition according to claim 5, **characterized in that** the K⁺ channel opener is selected from the group consisting of benzimidazole derivatives of general formula I 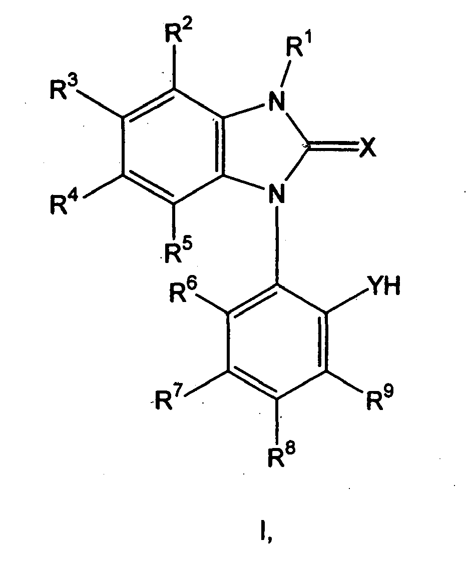 wherein
X represents O, S or NCN,
Y represents O or S,
R¹ represents hydrogen, NH₂ or branched or unbranched C₁₋₆-alkyl,
R², R³, R⁴, R⁵ are each independently selected from the group consisting of hydrogen, halogen, CF₃, NO₂, NH₂, OH, C₁₋₆-alkoxy, C(=O)-phenyl or SO₂NR^{A}R^{B}, wherein R^{A} and R^{B}, identical or different, represent H or C₁₋₆-alkyl,
R⁶ represents hydrogen or NO₂,
R⁷ represents hydrogen, halogen, phenyl, CF₃ or NO₂, or
R⁸ represents hydrogen or NO₂,
or
R⁶ and R⁷ or R⁷ and R⁸ together with the two bridging carbon atoms from the phenyl ring form a C₄₋₇ carbocyclic ring, which may be saturated, unsaturated or aromatic,
R⁹ is hydrogen, halogen, NO₂ or SO₂NR^{A}R^{B}, wherein R^{A} and R^{B}, identical or different represent hydrogen or C₁₋₆-alkyl,
optionally in the form of a corresponding salt, or a corresponding solvate thereof, and 6-Amino-1,2-dihydro-1-hydroxy-2-imino-4-piperidinopyrimidine (minoxidil), (R)-(-)-2-[4-(4-Methyl-6-oxo-1,4,5,6,-tetrahydropyridazin-3-yl)phenylhydrazono]propanedinitrile (levosimendan), N-[2-Amino-4-(4-fluorobenzylamino)phenyl]carbamic acid ethyl ester (retigabine), (-)-3-[5-oxo-2-(trifluoromethyl)-1,4,5,6,7,8-hexahydroquinolin-4(S)-yl]benzonitrile (ZD-0947), (3S, 4R)-3-Hydroxy-2,2-dimethyl-4-(2-oxopiperidin-1-yl)-N-phenyl-1-benzopyran-6-sulfonamide (KCO-912), (6-Chloro-3-(1-methylcyclopropylamino)-4H-thieno[3,2-e][1,2,4]thiadiazine-1,1-dioxide (NN-414), ABT-598, iptakalim hydrochloride, pinacidil, cromakalin, levcromakalin, aprikalim, N-(2-Hydroxyethyl)pyridine-3-carboxamide nitrate ester (nicorandil), (±)-(5-chloro-2-methoxyphenyl)-1 ,3-dihydro-3-fluoro-6-(trifluoromethyl)-2H-indol-2-one and ((3S)-(+)-(5-chloro-2-methoxyphenyl)-1,3-dihydro-3-fluoro-6-(trifluoromethyl)-2H-indol-2-one (BMS-204352).

7. Pharmaceutical composition according to one or more of claims 1-6, **characterized in that** the K⁺ channel opener is selected from the group consisting of NS-1619, NS-8, NS-1608, NS-0004 and BMS-204352, more preferably from the group consisting of NS-1619 and NS-8.

8. Pharmaceutical composition according to claim 7, **characterized in that** the K⁺ channel opener is NS-1619.

9. Pharmaceutical composition according to one or more of claims 1-8, **characterized in that** the phosphodiesterase type 5 inhibitor of component (C) is selected from the group consisting of sildenafil, vardenafil, tadalafil and dipyridamole, in each case optionally in form of a physiologically acceptable salt, or a corresponding solvate.

10. Pharmaceutical composition according to claim 9, **characterized in that** the phosphodiesterase type 5 inhibitor of component (C) is selected from the group consisting of sildenafil, sildenafil hydrochloride, sildenafil nitrate, sildenafil acetate, sildenafil hydrobromide, sildenafil lactate, sildenafil mesilate, sildenafil maleate, sildenafil fumarate, sildenafil succinate, sildenafil tatrate, sildenafil ascorbate and sildenafil citrate.

11. Pharmaceutical composition according to claim 10, **characterized in that** the phosphodiesterase type 5 inhibitor of component (C) is selected from the group consisting of sildenafil and sildenafil citrate.

12. Pharmaceutical composition according to one or more of claims 1-11 for once daily administration, **characterized in that** it comprises component (A) in an amount of 2.5 - 20 mg/kg, component (B) in an amount of 0.075 - 10 mg/kg and component (C) in an amount of 0.075 - 0.6 mg/kg, preferably component (A) in an amount of 5 - 15 mg/kg, component (B) in an amount of 0.15 - 7.5 mg/kg and component (C) in an amount of 0.15 - 0.45 mg/kg.

13. Pharmaceutical composition according to one or more of claims 1-12, **characterized in that** the combined intravenous administration of 10 mg/kg body weight of component (A), 0.3 mg /kg body weight of component (B) and 0.3 mg/kg body weight of component (C) causes an increase in intracavernosal pressure (ICP) in anesthetized diabetic rats of > 3000 area under curve, mm Hg x s, preferably of > 3500 area under curve, mm Hg x s, more preferably of > 3750 area under curve, mm Hg x s, yet more preferably of> 4000 area under curve, mm Hg x s, most preferably of > 4500 area under curve, mm Hg x s, in each case measured at a frequency of 3 Hz.

14. Medicament comprising a pharmaceutical composition according to one or more of claims 1-13 and optionally at least one auxiliary substance.

15. Medicament according to claim 14 for the prophylaxis and/or treatment of sexual disfunction in man and/or woman.

16. Medicament according to claim 15 for the prophylaxis and/or treatment of male sexual disfunction selected from the group consisting of erectile disfunction and impotence.

17. Medicament according to claim 14 for the prophylaxis and/or treatment of a disorder selected from the group consisting of hypertension, type I diabetes mellitus, type II diabetes mellitus, hypercholesterolemia, bladder instability, urinary incontinence, asthma, ischemic injury, ischemic insufficiency to the brain, cardiovascular diseases, preterm labor or for stopping labor preparatory to Caesarean delivery, alopecias, epilepsy, gastrointestinal disorders including ulcers and dyspepsia, spasms including gastrointestinal spasms, inflammatory diseases including gastrointestinal inflammation and cancer.

18. Use of a pharmaceutical composition according to one or more of claims 1-13 for the manufacture of a medicament for the prophylaxis and/or treatment of sexual disfunction in man and/or woman.

19. Use according to claim 18 for the manufacture of a medicament for the prophylaxis and/or treatment of male sexual disfunction selected from the group consisting of erectile disfunction and impotence.

20. Use of a pharmaceutical composition according to one or more of claims 1-13 for the manufacture of a medicament for the prophylaxis and/or treatment of a disorder selected from the group consisting of hypertension, type I diabetes mellitus, type II diabetes mellitus, hypercholesterolemia, bladder instability, urinary incontinence, asthma, ischemic injury, ischemic insufficiency to the brain, cardiovascular diseases, preterm labor or for stopping labor preparatory to Caesarean delivery, alopecias, epilepsy, gastrointestinal disorders including ulcers and dyspepsia, spasms including gastrointestinal spasms, inflammatory diseases including gastrointestinal inflammation and cancer.
